# EUROPEAN PATENT APPLICATION

(11) **EP 4 834 357 A1**
(43) Date of publication of application: **04.11.2026**
(21) Application number: 24910776.4
(22) Date of filing: 13.12.2024
(51) Int. Cl.: A61K 36/704, A61K 36/539, A61K 36/288, A61K 36/28, A61P 13/12

(54) **USE OF TRADITIONAL CHINESE MEDICINE IN PREPARING DRUG FOR PREVENTING AND TREATING NEPHRITIS**

(30) Priority: 25.12.2023 CN 202311798771
(71) Applicant: Tsing Hua De Ren Xi'An Xingfu Pharmaceutical Co. Ltd., Xi'an, Shaanxi 710043 (CN)
(72) Inventor: DU, Chengqiang, Shaanxi 710043 (CN)
(74) Representative: Schiweck Weinzierl Koch Patentanwälte Partnerschaft mbB
(86) International application number: PCT/CN2024/139109
(87) International publication number: WO 2025/139845

(57) **Abstract**

Use of a traditional Chinese medicine in preparing a drug for preventing and treating nephritis. The traditional Chinese medicine is prepared from Taraxacum officinale, Polygonum cuspidatum, Sonchus oleroceus L., and Herba Scutellariae baibatae. Danio rerio is adopted for modeling. Animal experiments prove that the traditional Chinese medicine can be used for treating nephritis caused by aristolochic acid,protecting kidney functions and/or ameliorating renal dysfunction edema, and specifically, promoting glomerular filtration.

## Description

The present application claims priority of Chinese patent application No. 202311798771.5 filed with the China National Intellectual Property Administration on December 25, 2023, entitled " Use of Traditional Chinese Medicine in Preparing Drug for Preventing and Treating Nephritis ", the entire contents of which are incorporated herein by reference.

### Technical Field

The present invention relates to the technical field of traditional Chinese medicines, and in particular to the use of a traditional Chinese medicine in the preparation of a medicament for the prophylaxis and/or treatment of nephritis.

### Background of the Invention

Chronic glomerulonephritis is currently the primary cause of chronic renal failure in China. The glomerular filtration rate (GFR) accurately reflects the filtration function of the kidney. Chronic glomerulonephritis is an immune-mediated inflammatory reaction occurring in the glomeruli. There are numerous causes of nephritis. Immune complexes may form when a patient has a bacterial/viral infection, or autoimmune dysfunction, or is exposed to allergy-related or drug-related factors. After immune complexes are deposited in the glomeruli, these complexes may induce thickening of the glomerular basement membrane, proliferation of glomerular mesangial cells, increase of mesangial matrix, or proliferation of capillary endothelial cells.

Currently, glucocorticoids are commonly used in the treatment of nephritis; however, they can only suppress immune responses and alleviate inflammation but are insufficient to comprehensively eliminate the multiple pathogenic factors responsible for damaging, for example the intrinsic cells of the glomeruli, thereby failing to achieve complete repair of these injured intrinsic glomerular cells. Moreover, glucocorticoids are associated with significant toxic side effects. Long-term use of glucocorticoids, while achieving therapeutic remission, leads to a considerable decline in immune function. For instance, Wang Li et al. conducted a statistical analysis of 199 cases of adverse reactions to dexamethasone reported in various domestic journals. Their finding indicated that adverse reactions to dexamethasone include systemic damage, central and peripheral nervous system damage, psychiatric symptoms, musculoskeletal system damage, skin and appendage lesions, visual impairment, general cardiovascular system damage, digestive system damage, respiratory system impairment, endocrine disturbances, reproductive system damage, metabolic and nutritional disorders, hepatobiliary system damage, platelet abnormalities and coagulation/bleeding abnormalities. Among these, anaphylactic shock should be considered a primary concern (Wang Li, et al., 'Literature Analysis of 199 Cases of Adverse Reactions to Dexamethasone').

Under the influence of these adverse effects, patients' conditions are prone to frequent relapse and become protracted and difficult to recover. Traditional Chinese Medicine (TCM) is based on syndrome differentiation and treatment. While treating nephritis, TCM can also preserve renal function and delay the progression of chronic renal failure. The TCM therapeutic approaches do not cause significant suffering to patients, are associated with minimal adverse effects, are convenient to administer, and address the condition at its root. Therefore, there is a need to develop therapeutic agents that can effectively treat nephritis while avoiding the aforementioned adverse effects or reducing their severity. Western pharmaceuticals are generally characterized by considerable adverse effects, long development cycles, and high development costs.

Consequently, identifying a TCM formulation capable of preventing and treating nephritis represents a promising and viable solution.

### Summary of the Invention

In view of the above, the technical problem to be solved by the present invention is to provide the use of a traditional Chinese medicine composition in the preparation of a medicament for the prophylaxis and/or treatment of nephritis. By utilizing the traditional Chinese medicine to treat nephritis, the present invention further achieves the effects of protecting renal function, delaying the progression of chronic renal failure, and fundamentally regulating the body.

The present invention provides a use of a traditional Chinese medicine in the preparation of a medicament for the prophylaxis and/or treatment of nephritis; wherein the traditional Chinese medicine is prepared from Taraxaci Herba, Polygoni Cuspidati Rhizome et Radix, Sonchi Arvensis Herba, and Scutellariae Barbatae Herba.

The present invention provides the traditional Chinese medicine for use in the treatment of nephritis. Experimental results indicate that it possesses renoprotective effects, specifically manifested as promoting glomerular filtration. The medicament is capable of delaying the progression of chronic renal failure and fundamentally regulates the body.

In the present invention, the nephritis is chronic glomerulonephritis.

In an embodiment of the present invention, the nephritis is nephritis induced by drug-induced injury.

In some embodiments, the drug is aristolochic acid.

In the present invention, the prophylaxis and/or treatment of nephritis comprises protecting renal function. In some embodiments, protecting renal function comprises promoting glomerular filtration.

In the present invention, the prophylaxis and/or treatment of nephritis comprises ameliorating edema associated with renal dysfunction.

In the present invention, the term "prophylaxis and/or treatment" is used, wherein the term "treatment" refers to a process carried out directly on a living human or animal body to eliminate the cause or pathological focus. The beneficial or desired clinical results of the treatment include, but are not limited to, alleviation of symptoms, reduction in disease severity, stabilization of disease state (i.e., non-worsening), delay or slowing of disease progression, amelioration or remission of the disease state, and remission (whether partial or complete), regardless of whether such results are detectable or undetectable.

In the present invention, the subjects for the prophylaxis and/or treatment include both subjects already suffering from a condition or disease and subjects susceptible to a condition or disease. When terms such as slow, alleviate, reduce, ameliorate, remit and the like, their meanings also encompass elimination, disappearance, non-occurrence and other such situations.

In the present invention, the subjects for the prophylaxis and/or treatment refer to living organisms that receive the treatment with the traditional Chinese medicine of the present invention, including mammals or non-mammals undergoing treatment for a disease or condition. Exemplary mammals include those of the groups Bovidea, Equidae, Ovidae, Suidae, Canidae, Felidae, Rodentia, and Primates. Preferably, the mammal is a human. In the examples, the efficacy is evaluated using a non-mammalian animal as an example, and the non-mammalian animal is specifically zebrafish.

In the present invention, the effective concentration for preventing and/or treating nephritis ranges from 0.061 µL/mL to 0.244 µL/mL. For example, it is 0.061 µL/mL, 0.122 µL/mL, or 0.244 µL/mL, which corresponds to a human dose of 21.65 mL to 86.62 mL.

The effective dose as described above refers to the amount of therapeutic agent that, when administered to cells, tissues or a subject, is effective in preventing or alleviating a disease or condition, or the progression thereof. An "effective amount" also refers to an amount of a compound sufficient to alleviate symptoms, for example, to treat, cure, prevent or alleviate a related medical condition, or to accelerate the treatment, cure, prevention, or alleviation of such conditions. When an active ingredient is administered alone to an individual, the therapeutically effective dose refers to that ingredient alone. When a combination is used, the therapeutically effective dose refers to the combined amount of active ingredients that produces the therapeutic effect, whether the ingredients are administered in combination, sequentially or simultaneously.

In the present invention, the traditional Chinese medicine is prepared from the following raw materials, in parts by mass: 372 parts of Taraxaci Herba, 372 parts of Polygoni Cuspidati Rhizome et Radix, 372 parts of Sonchi Arvensis Herba, and 186 parts of Scutellariae Barbatae Herba.

In some embodiments, the traditional Chinese medicine further comprises a pharmaceutically acceptable carrier or excipient. Specifically, the pharmaceutical composition is a solid preparation, aqueous decoction mixture, an injection, a preparation for external use, a spray, a liquid preparation, or a compound formulation.

In some embodiments, the traditional Chinese medicine is prepared by decoction with water.

In some specific embodiments, the preparation method of the traditional Chinese medicine comprises subjecting the raw materials to water decoction extraction, filtering the extract, centrifuging the filtrate, collecting the supernatant, adding stevioside and ethylparaben to the supernatant, heating the mixture to boiling, and then obtaining the traditional Chinese medicine.

The water decoction is performed twice: the first decoction is conducted with water in an amount of 5 to 15 times the mass of the raw materials and is boiled for 2 hours, and the second decoction is conducted with water in an amount of 4 to 10 times the mass of the raw materials and is boiled for 1 hour. Preferably, the first decoction uses water in an amount of 8 times the mass of the raw materials, and the second decoction uses water in an amount of 5 times the mass of the raw materials.

The present invention further provides a method for the prophylaxis and/or treatment of nephritis, comprising administering the traditional Chinese medicine as described above.

In the present invention, the traditional Chinese medicine may be administered by routes including, but not limited to, oral administration, administration by spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration by means of an implanted reservoir. Among these routes, oral, intraperitoneal or intravenous administration is preferred.

In the experimental protocol of the present invention, a zebrafish model is established, and animal experiments are conducted to demonstrate that the traditional Chinese medicine as described above can treat nephritis and has a protective effect on renal function, specifically manifested as promoting glomerular filtration. Moreover, the traditional Chinese medicine exhibits good safety and is not expected to cause severe adverse effects.

### Brief Description of the Drawings

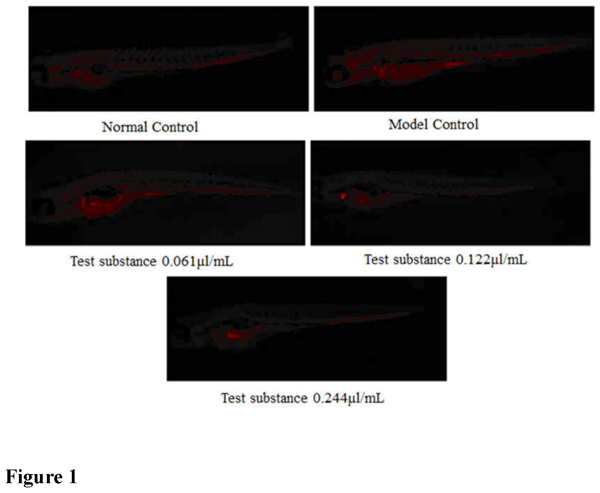

Figure 1 shows a representative image of whole-body fluorescence intensity of zebrafish after sample treatment.

### Detailed Description of the Invention

The present invention provides the use of the traditional Chinese medicine in the preparation of a medicament for the prophylaxis and/or treatment of nephritis. Those skilled in the art can implement the invention by referring to the disclosure herein and appropriately modifying process parameters. It should be particularly noted that all similar substitutions and modifications are obvious to those skilled in the art and are deemed to be included in the present invention. The methods and uses of the present invention have been described through preferred embodiments, and relevant personnel will obviously be able to make modifications or appropriate changes and combinations to the methods and uses herein without departing from the content, spirit and scope of the present invention, so as to realize and apply the technology of the present invention.

The present invention provides the use of a traditional Chinese medicine in the prophylaxis and/or treatment of nephritis. The traditional Chinese medicine used in the present invention is prepared from Taraxaci Herba, Polygoni Cuspidati Rhizome et Radix, Sonchi Arvensis Herba, and Scutellariae Barbatae Herba, and it has a protective effect on renal function. Renal function can be divided into glomerular function and tubular function. Glomerular function is primarily assessed by evaluating the glomerular filtration rate, which may be clinically reflected by blood urea nitrogen and serum creatinine levels, or accurately assessed by calculating the endogenous creatinine clearance. Tubular function refers to the reabsorption and excretion functions of the renal tubules. In clinical practice, urinary NAG is commonly used to reflect whether tubular injury is present. In addition, tubular function can also be evaluated by urinary β2-microglobulin, urinary retinol-binding protein, urine specific gravity, urine osmolality, and the like. In the present invention, it is demonstrated through the detection of the glomerular filtration rate that the traditional Chinese medicine has a protective effect on renal function, particularly in a renal dysfunction edema model, thereby proving that the traditional Chinese medicine has a prophylactic and/or therapeutic effect on nephritis.

The traditional Chinese medicine screened and obtained by the present invention is safer. When used for treating nephritis, it has fewer adverse effects compared with the commonly used drug dexamethasone, and is not expected to cause severe adverse effects, such as systemic damage, anaphylactic shock, central and peripheral nervous system damage, psychiatric symptoms, musculoskeletal system damage, skin and its appendages damage, visual impairment, general cardiovascular system damage, digestive system damage, respiratory system damage, endocrine disorders, reproductive system damage, metabolic and nutritional disorders, hepatobiliary system damage, platelet abnormalities, or abnormalities in blood coagulation and bleeding.

In the example of the present invention, only one case in which a traditional Chinese medicine composition is used as the test substance is described. Beyond this, numerous attempts were made during the research and development process, such as using different Chinese medicinal materials, proprietary Chinese medicines, reagents, or employing different extraction methods. However, these experiments revealed that, whether using individual Chinese medicinal materials such as Taraxaci herba alone for testing, using other proprietary Chinese medicines, or using a composition containing Taraxaci herba as one of the components, the protective effect on renal function was inferior to that of the traditional Chinese medicine prepared from Taraxaci Herba, Polygoni Cuspidati Rhizome et Radix, Sonchi Arvensis Herba, Scutellariae Barbatae Herba. The specific experimental results are not reiterated herein. These results demonstrate that Taraxaci Herba, Polygoni Cuspidati Rhizome et Radix, Sonchi Arvensis Herba, Scutellariae Barbatae Herba cooperate with each other, are indispensable. With an appropriate ratio, they can produce a synergistic effect, thereby exerting a more significant renoprotective effect.

All test materials used in the present invention are common commercially available products and can be purchased from the market.

The present invention is further described below in conjunction with examples.

### Example: Renoprotective Effect

### 1. Test Materials

### 1.1. Sample Preparation Information

**Traditional Chinese medicine decoction**: The traditional Chinese medicine involved in the examples of the present invention is prepared from Taraxaci Herba (monarch herb), Polygoni Cuspidati Rhizome et Radix (minister herb), Sonchi Arvensis Herba (assistant and guide herb) and Scutellariae Barbatae Herba(assistant and guide herb). The preparation method comprises: taking 372g of Taraxaci Herba, 372g of Polygoni Cuspidati Rhizome et Radix, 372g of Sonchi Arvensis Herba, and 186g of Scutellariae Barbatae Herba, adding water and decocting twice, wherein the first decoction is conducted with water in an amount of 8 times the mass of the raw materials and is boiled for 2 hours, and the second decoction is conducted with water in an amount of 5 times the mass of the raw materials and is boiled for 1 hour, filtering the decoction, concentrating the filtrate under reduced pressure, combining the concentrates, centrifuging, filtering, adding 1.5 g of Stevioside and 0.5 g of ethylparaben to the filtrate, heating to boiling, and making up to 1000 mL to obtain the decoction.

Test substance: solvent was 1 × E3 culture medium, and the above decoction was mixed with E3 culture medium before use.

### 1.2. Experimental Animals

All zebrafish were maintained in aquaculture water at 28°C (water quality: 200 mg of instant sea salt per 1 L of reverse osmosis water, conductivity of 450-550 µS/cm; pH 6.5-8.5; hardness of 50-100 mg/L CaCO₃). The zebrafish were bred and provided by the company's fish rearing center. The license number for the use of experimental animals is SYXK (Zhejiang) 2022-0004. The husbandry and management complied with the requirements of international AAALAC accreditation (accreditation number: 001458). The IACUC ethics approval number was IACUC-2023-7687-01.

### 1.3. Instruments, Consumables and Reagents

Dissecting microscope (SZX7, OLYMPUS, Japan); CCD camera (VertA1, Shanghai Tushi Vision Technology Co., Ltd., China); motorized focusing continuous zoom fluorescence microscope (AZ100, Nikon, Japan); microinjector (IM300, Narishige, Japan); needle puller (PC-10, Narishige, Japan); precision electronic balance (CP214, OHAUS, USA); 6-well plate (Zhejiang Bioland Biotechnology Co., Ltd., China).

Methylcellulose (Lot No. C2004046, Shanghai Aladdin Biochemical Technology Co., Ltd., China); dimethyl sulfoxide (DMSO, Lot No. BCCD8942, Sigma, Switzerland); aristolochic acid (Lot No. E2015060, Shanghai Aladdin Biochemical Technology Co., Ltd., China); Dextran tetramethylrhodamine (Lot No. 923446, Molecular Probes, USA).

### 2. Detection Methods

### 2.1. Determination of Maximum Tested Concentration (MTC)

Wild-type AB zebrafish at 2 days post-fertilization (2 dpf) were randomly selected and placed into 6-well plates, with 30 zebrafish treated per well (experimental group). The zebrafish were exposed to the test substances dissolved in water (concentrations as shown in Table 1-1). A normal control group and a model control group were also established. The volume per well was 3 mL. Except for the normal control group, all other experimental groups were administered aristolochic acid dissolved in water to establish a zebrafish renal dysfunction edema model. After treatment at 28°C for 2 days, the MTC of the samples for the model zebrafish was determined.

### 2.2. Renoprotective Effect (Glomerular Filtration)

Wild-type AB zebrafish strain at 2 days post-fertilization (dpf) were randomly selected and placed into 6-well plates, with 30 zebrafish treated per well (experimental group). The test substances were dissolved in water and administered to the zebrafish (concentrations as shown in Table 1-2). A normal control group and a model control group were also established. The volume per well was 3 mL. Except for the normal control group, all other experimental groups were exposed to aqueous aristolochic acid to establish a zebrafish model of renal dysfunction- associated edema. After treatment at 28°C for 1 day, each experimental group was intravenously injected with the fluorescent marker (Dextran tetramethylrhodamine). After continued treatment at 28°C for another day, 10 zebrafish were randomly selected from each experimental group and photographed under a fluorescence microscope. The NIS-Elements D 3.20 advanced image processing software was used to analyze and collect data, and the whole-body fluorescence intensity of the zebrafish was analyzed. The statistical analysis results of this indicator were used to evaluate the renoprotective effect (glomerular filtration) of the samples. The statistical results were expressed as mean ± SE. Statistical analysis was performed using SPSS 26.0 software, and p < 0.05 indicated that the difference was statistically significant.

### 3. Results

### 3.1. MTC

Under the conditions of the present experiment, the maximum tested concentration(MTC)of the test substance for renoprotective effect was 0.244 µL/mL. See Table 1-1 for details.

**Table 1-1. Results of the concentration range-finding experiment for the renoprotective effect of the test substance (n = 30)**

| Group | Concentration (µL/mL) | No. of Death (fish) | Mortality (%) | Phenotype | Human Equivalent Dose (mL) |
|---|---|---|---|---|---|
| Normal Control | - | 0 | 0 | No obvious abnormalities | |
| Model Control | - | 0 | 0 | Renal edema | |
| Test Substance | 0.061 | 0 | 0 | Comparable to the model control group | 21.65 |
| | 0.122 | 0 | 0 | Comparable to the model control group | 43.31 |
| | 0.244 | 0 | 0 | Comparable to the model control group | 86.62 |
| | 0.488 | 0 | 0 | Worse than the model control group | 173 |
| | 0.977 | 5 | 17 | - | 346 |

### 3.2. Evaluation of Renoprotective Effect (Glomerular Filtration)

Under the conditions of the present experiment, the test substance exhibited a renoprotective effect, specifically manifested as the promotion of glomerular filtration. See Table 1-2 and Figure 1.

**Table 1-2. Results of the renoprotective effect evaluation (glomerular filtration) of the test substance (n = 10)**

| Group | Concentration (µL/mL) | Whole-body fluorescence intensity (pixel value, mean ± SE) |
|---|---|---|
| Normal Control | - | 405609±24900*** |
| Model Control | - | 600535±37985 |
| Test Substance | 0.061 | 561919±39955 |
| | 0.122 | 489486±20169 |
| | 0.244 | 444244±21684* |

Compared with the group of model control, *p < 0.05, ***p < 0.001

The above are merely preferred embodiments of the present invention. It should be noted that for those skilled in the art, several improvements and modifications can be made without departing from the principle of the present invention, and such improvements and modifications should also be regarded as falling within the protection scope of the present invention.

## Claims

1. Use of a traditional Chinese medicine in the preparation of a medicament for the prophylaxis and/or treatment of nephritis; wherein the traditional Chinese medicine is prepared from Taraxaci Herba, Polygoni Cuspidati Rhizome et Radix, Sonchi Arvensis Herba, and Scutellariae Barbatae Herba.

2. The use according to claim 1, **characterized in that** the nephritis is chronic glomerulonephritis.

3. The use according to claim 2, **characterized in that** the nephritis is nephritis induced by drug-induced injury.

4. The use according to claim 3, **characterized in that** the drug is aristolochic acid.

5. The use according to claim 1, **characterized in that** the prophylaxis and/or treatment of nephritis comprises protecting renal function and/or ameliorating edema associated with renal dysfunction.

6. The use according to claim 5, **characterized in that** protecting renal function comprises promoting glomerular filtration.

7. The use according to claim 1, **characterized in that** the effective concentration for the prophylaxis and/or treatment of nephritis is 0.061 µL/mL to 0.244 µL/mL, corresponding to a human dose of 21.65 mL to 86.62 mL.

8. The use according to any one of claims 1 to 7, **characterized in that** the traditional Chinese medicine is prepared from the following raw materials, in parts by mass:
372 parts of Taraxaci Herba, 372 parts of Polygoni Cuspidati Rhizome et Radix, 372 parts of Sonchi Arvensis Herba, and 186 parts of Scutellariae Barbatae Herba.

9. The use according to claim 8, **characterized in that** the preparation method of the traditional Chinese medicine comprises subjecting the raw materials to water decoction extraction, filtering the extract, centrifuging the filtrate, collecting the supernatant, adding stevioside and ethylparaben to the supernatant, heating the mixture to boiling, and then obtaining the traditional Chinese medicine.

10. A method for the prophylaxis and/or treatment of nephritis, **characterized in that** it comprises administering a traditional Chinese medicine;
wherein the traditional Chinese medicine is prepared from the following raw materials in parts by mass:
372 parts of Taraxaci Herba, 372 parts of Polygoni Cuspidati Rhizome et Radix, 372 parts of Sonchi Arvensis Herba, and 186 parts of Scutellariae Barbatae Herba.
